# EUROPÄISCHE PATENTSCHRIFT

(11) **EP 1 186 288 B1**
(45) Veröffentlichungstag und Bekanntmachung des Hinweises auf die Patenterteilung: **03.06.2009**
(21) Anmeldenummer: 01117897.7
(22) Anmeldetag: 24.07.2001
(51) Int. Cl.: A61K 8/49, A61K 8/895, A61Q 17/04

(54) **Kosmetische und dermatologische Lichtschutzformulierungen mit einem Gehalt an unsymmetrisch substituierten Triazinderivaten und Organsiloxanen**
Cosmetic and dermatologic sunscreen compositions containing asymmetrically substituted triazine derivatives and organosiloxanes
Compositions cosmétiques ou dermatologiques photoprotectrices contenant des dérivés de triazine substitués asymétriquement et des organosiloxanes

(30) Priorität: 02.08.2000 DE 10037558
(43) Veröffentlichungstag der Anmeldung: 13.03.2002
(73) Patentinhaber: Beiersdorf AG, 20253 Hamburg (DE)
(72) Erfinder: Müller, Anja, 23843 Rümpel (DE); Grund, Wiebke, 21244 Buchholz (DE)

(56) Entgegenhaltungen:
- EP-A- 0 998 906
- DE-A- 19 841 798
- US-A- 5 882 632

## Beschreibung

Die vorliegende Erfindung betrifft kosmetische und dermatologische Lichtschutzzubereitungen, insbesondere betrifft sie sandabweisende kosmetische und dermatologische Lichtschutzzubereitungen.

Die schädigende Wirkung des ultravioletten Teils der Sonnenstrahlung auf die Haut ist allgemein bekannt. In Abhängigkeit von ihrer jeweiligen Wellenlänge haben die Strahlen verschiedene Wirkungen auf das Organ Haut: Die sogenannte UV-C-Strahlung mit einer Wellenlänge, die kleiner als 290 nm ist, wird von der Ozonschicht in der Erdatmosphäre absorbiert und hat daher keine physiologische Bedeutung. Dagegen verursachen Strahlen im Bereich zwischen 290 nm und 320 nm, dem sogenannten UV-B-Bereich, ein Erythem, einen einfachen Sonnenbrand oder sogar mehr oder weniger starke Verbrennungen. Als ein Maximum der Erythemwirksamkeit des Sonnenlichtes wird der engere Bereich um 308 nm angegeben.

Zum Schutz gegen UV-B-Strahlung sind zahlreiche Verbindungen bekannt, bei denen es sich beispielsweise um Derivate des 3-Benzylidencamphers, der 4-Aminobenzoesäure, der Zimtsäure, der Salicylsäure, des Benzophenons, des 2-Phenylbenzimidazols sowie des s-Triazins handelt.

Man hat lange Zeit fälschlicherweise angenommen, daß die langwellige UV-A-Strahlung mit einer Wellenlänge zwischen 320 nm und 400 nm nur eine vernachlässigbare biologische Wirkung aufweist. Inzwischen ist allerdings durch zahlreiche Studien belegt, daß UV-A-Strahlung im Hinblick auf die Auslösung photodynamischer, speziell phototoxischer Reaktionen und chronischer Veränderungen der Haut weitaus gefährlicher als UV-B-Strahlung ist. Auch kann der schädigende Einfluß der UV-B-Strahlung durch UV-A-Strahlung noch verstärkt werden.

So ist es u.a. erwiesen, daß selbst die UV-A-Strahlung unter ganz normalen Alltagsbedingungen ausreicht, um innerhalb kurzer Zeit die Collagen- und Elastinfasem zu schädigen, die für die Struktur und Festigkeit der Haut von wesentlicher Bedeutung sind. Hierdurch kommt es zu chronischen lichtbedingten Hautveränderungen - die Haut "altert" vorzeitig. Zum klinischen Erscheinungsbild der durch Licht gealterten Haut gehören beispielsweise Falten und Fältchen sowie ein unregelmäßiges, zerfurchtes Relief. Ferner können die von lichtbedingter Hautalterung betroffenen Partien eine unregelmäßige Pigmentierung aufweisen. Auch die Bildung von braunen Flecken, Keratosen und sogar Karzinomen bzw. malignen Melanomen ist möglich. Eine durch die alltägliche UV-Belastung vorzeitig gealterte Haut zeichnet sich außerdem durch eine geringere Aktivität der Langerhanszellen und eine leichte, chronische Entzündung aus.

Etwa 90 % der auf die Erde gelangenden ultravioletten Strahlung besteht aus UV-A-Strahlen. Während die UV-B-Strahlung in Abhängigkeit von zahlreichen Faktoren stark variiert (z. B. Jahres- und Tageszeit oder Breitengrad), bleibt die UV-A-Strahlung unabhängig von jahres- und tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Hornschicht zurückgehalten werden.

Es ist daher von grundsätzlicher Wichtigkeit, daß kosmetische und dermatologische Lichtschutzzubereitungen sowohl gegen UV-B- als auch gegen UV-A-Strahlung ausreichenden Schutz bieten.

Im allgemeinen ist das Lichtabsorptionsverhalten von Lichtschutzfiltersubstanzen sehr gut bekannt und dokumentiert, zumal in den meisten Industrieländem Positivlisten für den Einsatz solcher Substanzen existieren, welche recht strenge Maßstäbe an die Dokumentation anlegen.

Die Einsatzkonzentration bekannter als Feststoff vorliegender Lichtschutzfiltersubstanzen ist allerdings häufig - gerade in Kombination mit anderen zu lösenden Substanzen - begrenzt. Es bereitet daher gewisse formulierungstechnische Schwierigkeiten, höhere Lichtschutzfaktoren bzw. UV-A-Schutzleistung zu erzielen.

Von verschiedenen Autoren wurden UV-Filtersubstanzen vorgestellt, welche das Strukturmotiv 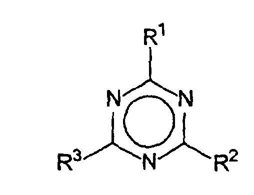 aufweisen.

Hinsichtlich der C₃-Achse des Triazingrundkörpers dieser Verbindungen sind sowohl symmetrische Substitution wie auch unsymmetrische Substitution denkbar. In diesem Sinne symmetrisch substituierte s-Triazine weisen drei gleiche Substituenten R¹ R² und R³ auf, während unsymmetrisch substituierte s-Triazinderivate demzufolge unterschiedliche Substituenten aufweisen, wodurch die C₃-Symmetrie zerstört wird. Im Sinne der vorliegenden Erfindung wird als "unsymmetrisch" stets unsymmetrisch hinsichtlich der C₃-Achse des Triazingrundkörpers verstanden, es sei denn, etwas anderes wäre ausdrücklich erwähnt.

DE-19841798 offenbart lichtschutzwirksame kosmetische Zubereitungen, die unsymmetrisch substituierte Triazine und Polymerfilter enthalten. US-5882632 und EP-998906 offenbaren lichtschutzwirksame kosmetische Zubereitungen, die Organosiloxanfilter und Triazine enthalten.

Um einen optimalen UV-Schutz zu gewährleisten, müssen UV-Filtersubstanzen selbstverständlich in gelöster Form vorliegen. Unsymmetrisch substituierte Triazinderivate zeichnen sich an sich durch gute Lichtschutzwirkung aus. Ihr Hauptnachteil liegt allerdings darin, daß sie schlecht in üblichen Ölkomponenten löslich sind. Dementsprechend lassen sich in bekannten Lösungsmitteln nur bis zu maximal 15 Gew.-% dieser Verbindungen lösen, was im Regelfall einer Konzentration von etwa 1 bis 1,5 Gew.-% gelöster (= aktiver) Filtersubstanz in der gesamten kosmetischen oder dermatologischen Zubereitung entspricht.

Ein Nachteil des Standes der Technik ist dementsprechend, daß mit diesen Filtersubstanzen in der Regel nur vergleichsweise niedrige Lichtschutzfaktoren erreicht werden konnten, da ihre Löslichkeit oder Dispergierbarkeit in den Formulierungen zu gering ist, d. h. sie lassen sich nicht oder nur schwer in befriedigender Weise in solche Formulierungen einarbeiten.

Selbst wenn grundsätzlich auch bei begrenzter Löslichkeit ein gewisser UV-Schutz erreicht werden kann, tritt häufig ein anderes Problem auf, die Rekristallisation. Insbesondere schlecht lösliche Substanzen rekristallisieren vergleichsweise schnell, was durch Temperaturschwankungen oder andere Einflüsse hervorgerufen werden kann. Unkontrollierte Rekristallisation eines wesentlichen Zubereitungsbestandteiles wie eines UV-Filters hat aber äußerst nachteilige Auswirkungen auf die Eigenschaften der gegebenen Zubereitung und - nicht zuletzt - auf den angestrebten Lichtschutz.

Es war Aufgabe der vorliegenden Erfindung, auf einfache Weise zu Zubereitungen zu gelangen, welche sich durch einen erhöhten Gehalt an unsymmetrisch substituierte Triazinderivate und einen demensprechend hohen Lichtschutzfaktor auszeichnen.

Ein weiterer Nachteil des Standes der Technik ist, daß übliche Lichtschutzformulierungen einen meist klebrigen Film auf der Haut hinterlassen. Dies hat z. B. bei der Anwendung solcher Produkte an einem Sandstrand zur Folge, daß der Sand am Körper haften bleibt, was vom Anwender als unangenehm empfunden wird und im schlimmsten Fall dazu führen kann, daß das Sonnenschutzmittel zu wenig oder gar nicht mehr verwendet wird. Da am Meer meist ein mehr oder weniger starker Wind herrscht, tritt dieser Nachteil in der Regel selbst dann auf, wenn der Körper gar nicht direkt mit dem Sand in Berührung kommt - beispielsweise beim Sonnenbaden auf einem Liegestuhl - da auch der im Wind herumwirbelnde Sandstaub auf den eingecremten Hautpartien haften bleibt.

Eine weitere Aufgabe der vorliegenden Erfindung war daher, Lichtschutzformutierungen zu finden, nach deren Anwendung kein Sand auf der eingecremten Haut kleben bleibt, die also dementsprechend als sandabweisend zu bezeichnen sind.

Es war überraschend und für den Fachmann nicht vorauszusehen, daß lichtschutzwirksame Wirkstoffkombinationen aus
(a) einer oder mehreren UV-Filtersubstanzen, gewählt aus der Gruppe der unsymmetrisch substituierten Triazinderivate gemäß Anspruch 1 und
(b) einem oder mehreren Organosiloxanen gemäß Anspruch 1. den Nachteilen des Standes der Technik abhelfen.

Die Organosiloxane werden als Lösungsmittel, Lösungsvermittler, Solubilisator oder Stabilisator für unsymmetrisch substituierte Triazinderivate verwendet.

Die Organosiloxane werden verwendet zur Erzielung oder Erhöhung der Löslichkeit unsymmetrisch substituierter Triazinderivate in
(a) entweder einer isolierten Ölkomponente oder
(b) in mindestens einer Ölphase eines dispersen Zwei- oder Mehrphasensystem, welches zusätzlich eine oder mehrere Wasserphasen enthalten kann.

Organosiloxane im Sinne der vorliegenden Erfindung sind Subtanzen, welche sich durch die Strukturformel (I) auszeichnen:

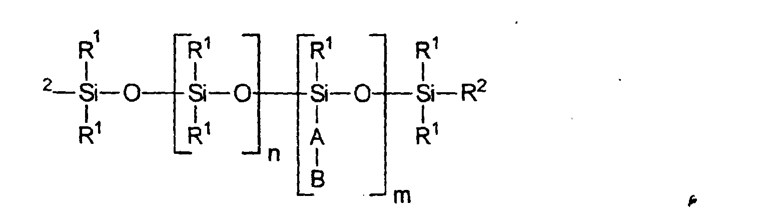

Derartige Substanzen zeichnen sich in der Regel durch eine gewisse UV-Filterleistung aus.

Ein besonders vorteilhaftes Organosiloxan im Sinne der vorliegenden Erfindung wird durch die folgende Strukturformel charakterisiert:

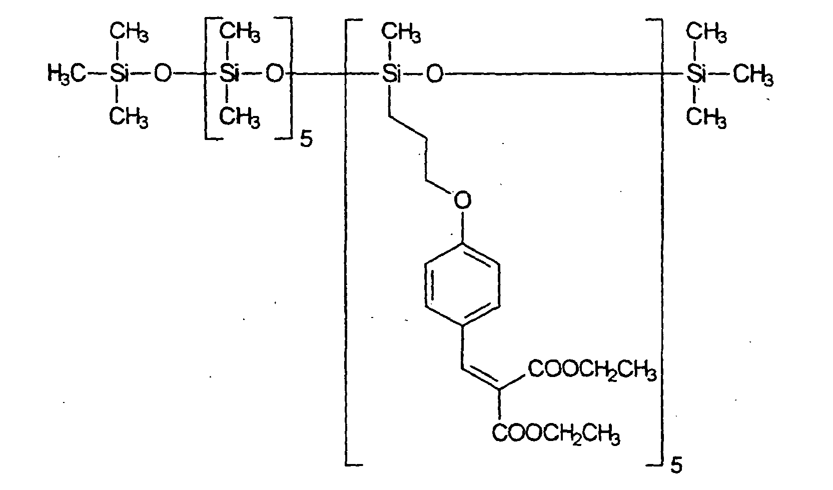

Die Organosiloxane sind soche, die sich durch die lineare Strukturformel (I) auszeichnen, wobei
◆ R¹ eine Methylgruppe und
◆ R² eine Methylgruppe oder die Gruppe A-B darstellen,
◆ die Gruppe A-B eine der beiden folgenden Strukturen hat: 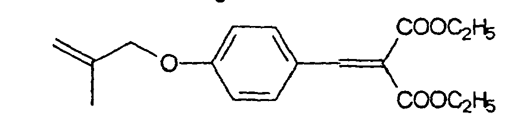 oder 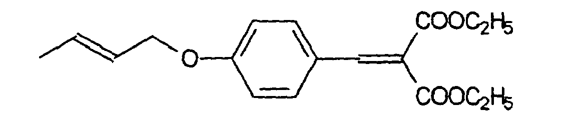 mit den Maßgaben, daß R² der Gruppe A-B entspricht, wenn m = 0 ist, und daß R² eine Methylgruppe ist, wenn m zwischen 1 und 20 liegt.

Erfindungsgemäß ganz besonders bevorzugt aus dieser Gruppe ist das α-(Trimethylsilyl)-ω-[((trimethylsilyl)-oxy-[poly-[oxy-[dimethyl-] und 6 % Methyl-[2-[p-[2,2-bis-(ethoxycarbonyl)-vinyl]phenoxy]-1-methylenethyl] und 1,5 % Metlrlyl-[3-[p-[2,2-bis-(ethoxycarbanyl)vinyl]-phenoxy]-propenyl] und 0,1% bis 0,4 % Methylhydrogen]silylene] (mit n ungefähr gleich 60), welches beispielsweise unter der Handelsbezeichnung Parsol® SLX bei der Fa. Givaudan erhältlich ist. Für diese Substanz ist der INCI-Name "Dimethicone-diethylbenzalmalonate" vorgesehen.

Es ist erfindungsgemäß bevorzugt, die lichtschutzwirksame Wirkstoffkombination in dispersen Zwei- oder Mehrphasensystemen einzusetzen, welche neben einer oder mehreren Ölphasen zusätzlich eine oder mehrere Wasserphasen enthalten können. Besonders bevorzugt sind kosmetische oder dermatologische Emulsionen, die die erfindungsgemäße Wirkstoffkombination enthalten, beispielsweise W/O-, O/W-, W/O/W- oder O/W/O-Emulsionen. Solche Emulsionen können vorzugsweise auch eine Mikroemulsion, eine Pickering-Emulsion oder eine sprühbare Emulsion sein. Es kann aber auch von Vorteil sein, wenn die erfindungsgemäßen Zubereitungen eine Lösung, eine Hydrodispersion, ein Aerosol, einen Schaum oder auch einen Stift darstellen.

Kosmetische und dermatologische Zubereitungen, welche die erfindungsgemäße Wirkstoffkombination enthalten, zeichnen sich überaschender Weise dadurch aus, daß sie sandabweisend sind.

Gegenstand der Erfindung sind daher auch
sandabweise, kosmetische oder dermatologische Lichtschutzzubereitungen, dadurch gekennzeichnet, daß sie
a) eine oder mehrere UV-Filtersubstanzen, gewählt aus der Gruppe der unsymmetrisch substituierten Triazinderivate gemäß Anspruch 1 und
b) ein oder mehrere Organosiloxane gemäß Anspruch 1
enthalten.

Die erfindungsgemäßen Zubereitungen sind in jeglicher Hinsicht überaus befriedigende Präparate, die erstaunlicherweise hervorragende kosmetische Eigenschaften zeigen, auf der Haut keinen schmierigen oder klebrigen Eindruck hinterlassen und sich durch eine ausgezeichnete Hautverträglichkeit auszeichnen.

Die vorliegende Erfindung betrifft kosmetische oder dermatologische Formulierungen mit einem Gehalt an mindestens einem unsymmetrisch substituierten s-Triazin gemäß Anspruch 1. In der EP-A-775 698 werden unsymmetrisch substituierte 5-Triazine der Strukturen: 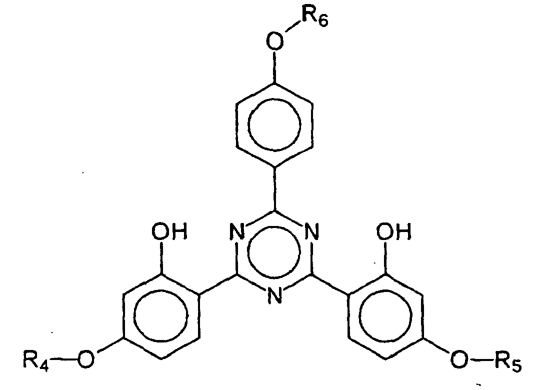 und/oder 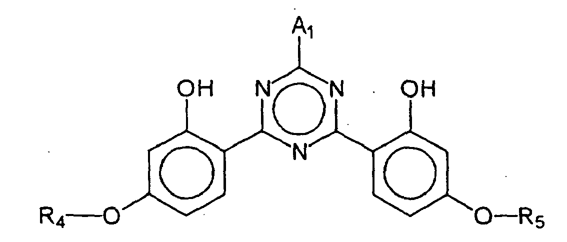 beschrieben. R₄ und R₅ werden vorteilhaft aus der Gruppe der verzweigten und unverzweigten Alkylgruppen von 1 bis 18 Kohlenstoffatomen gewählt. Auch können die Alkylgruppen wiederum vorteilhaft mit Silyloxygruppen substituiert sein.

A₁ stellt vorteilhaft einen substituierten homo- oder heterocyclischen aromatischen Fünfring oder Sechsring dar.

In der vorliegenden Erfindung werden die unsymmetrisch substituierten s-Triazin-Verbindungen ausgewählt aus der Gruppe 2,4-Bis-{[4-(2-ethyl-hexyloxy)-2-hydroxy]-phenyl}-6-(4-methoxyphenyl)-1,3,5-triazin (INCl: Aniso Triazin), welches unter der Handelsbezeichnung Tinosorb® S bei der CIBA-Chemikalien GmbH erhältlich und durch folgende Struktur gekennzeichnet ist: 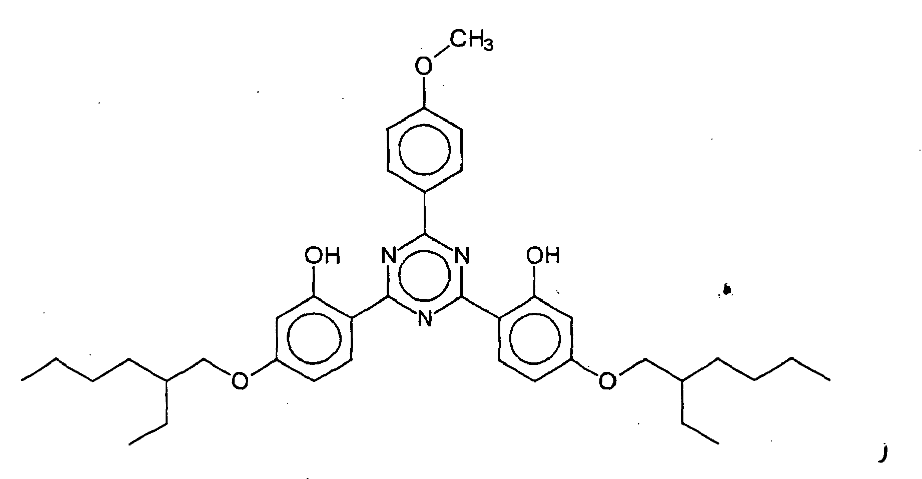 2,4-Bis-{[4-(3-sulfonato)-2-hydroxy-propyloxy)-2-hydroxy]-phenyl}-6-(4-methoxyphenyl)-1,3,5-triazin Natriumsalz, welches durch folgende Struktur gekennzeichnet ist: 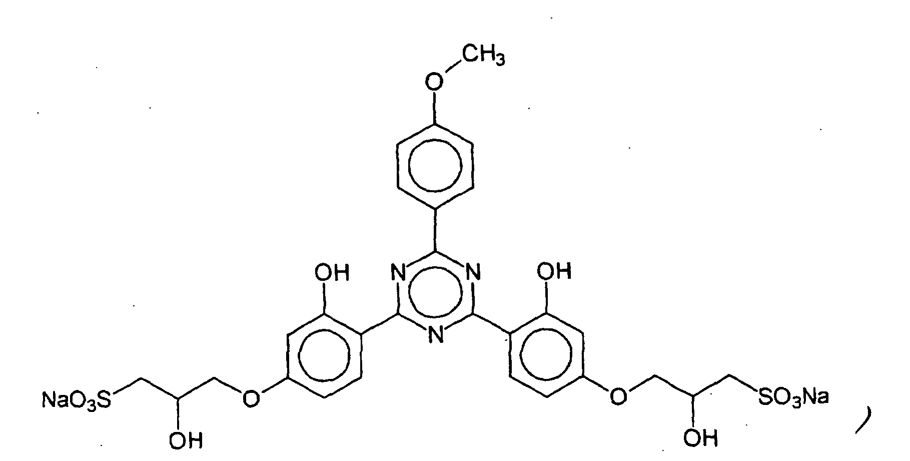 2,4-Bis-{[4-(3-(2-propyloxy)-2-hydroxy-propyloxy)-2-hydroxy]-phenyl}-6-(4-methoxyphenyl)-1,3,5-triazin, welches durch folgende Struktur gekennzeichnet ist: 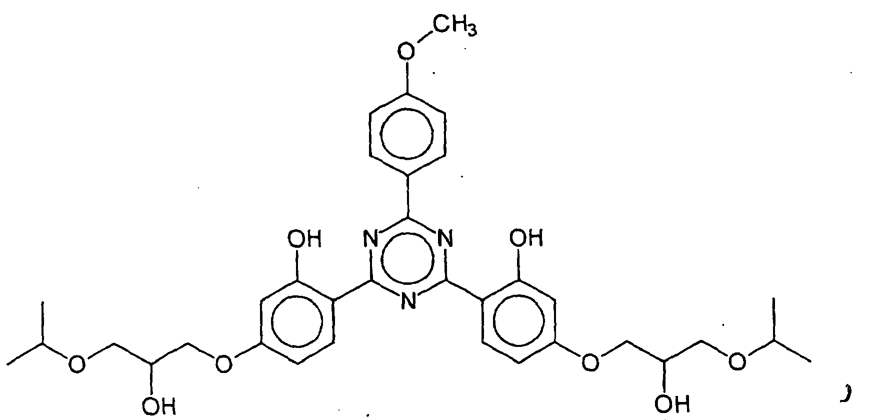 2,4-Bis-{[4-(2-ethyl-hexyloxy)-2-hydroxy]-phenyl}-6-[4-(2-methoxyethyl-carboxyl)-phenylamino]-1,3,5-triazin, welches durch folgende Struktur gekennzeichnet ist: 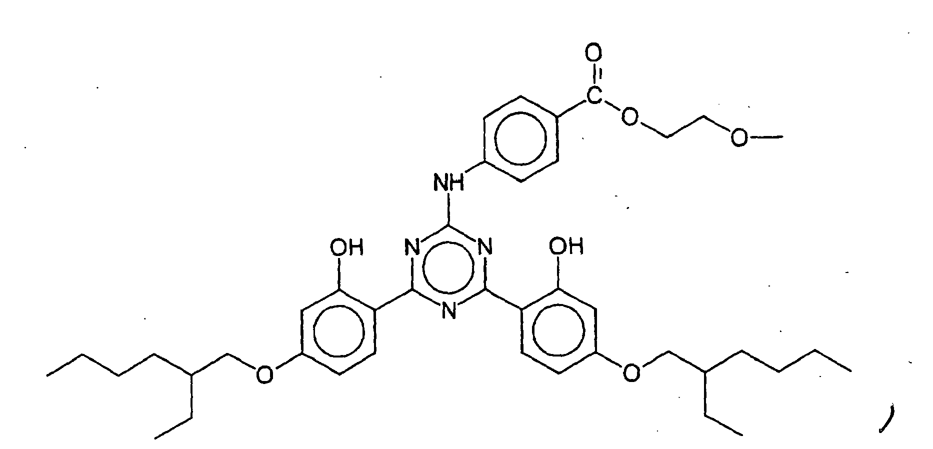 2,4-Bis-{[4-(3-(2-propyloxy)-2-hydroxy-propyloxy)-2-hydroxy]-phenyl}-6-[4-(ethylcarboxyl)-phenylamino]-1,3,5-triazin, welches durch folgende Struktur gekennzeichnet ist: 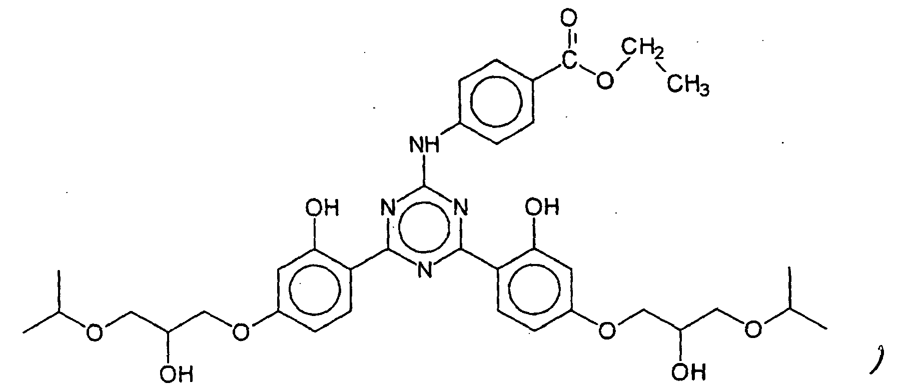 2,4-Bis-{[4-(2-ethyl-hexyloxy)-2-hydroxy]-phenyl}-6-(1-methyl-pyrrol-2-yl)-1,3,5-triazin, welches durch folgende Struktur gekennzeichnet ist: 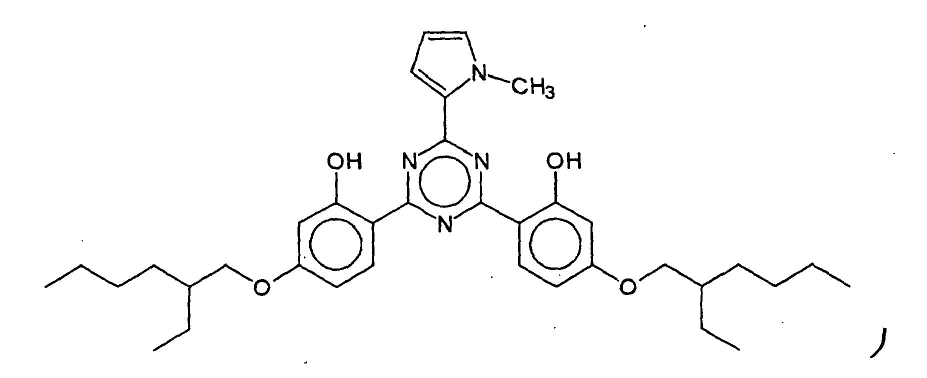 2,4-Bis-{[4-tris(trimethylsiloxy-silylpropyloxy)-2-hydroxy]-phenyl}-6-(4-methoxyphenyl)-1,3,5-triazin, welches durch folgende Struktur gekennzeichnet ist: 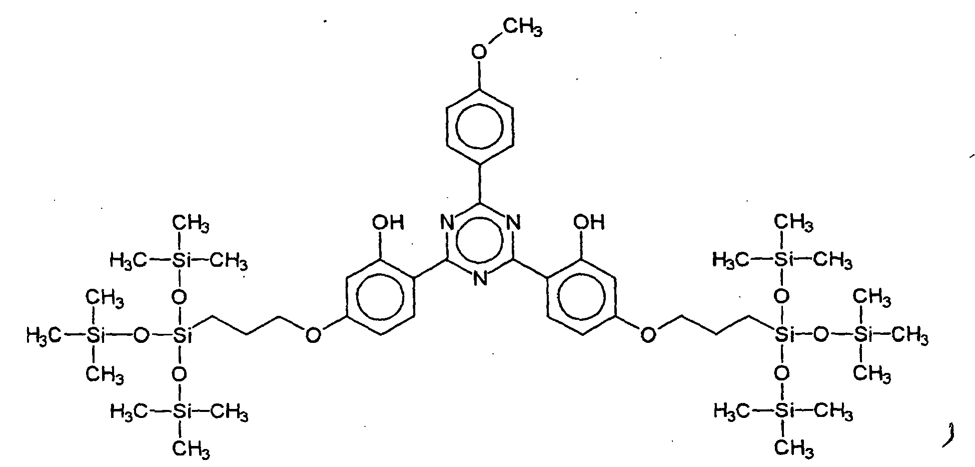 2,4-Bis-{[4-(2-methylpropenyloxy)-2-hydroxy]-phenyl}-6-(4-methoxyphenyl)-1,3,5-triazin, welches durch folgende Struktur gekennzeichnet ist: 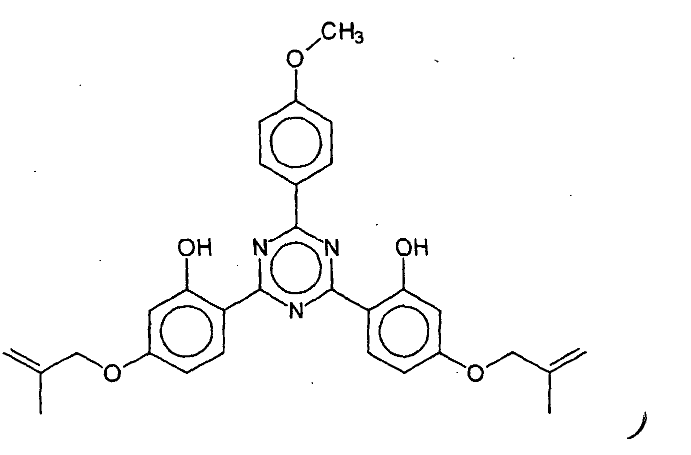 2,4-Bis-{[4-(1',1',1',3',5',5',5'-Heptamethylsiloxy-2-methyl-propyloxy)-2-hydroxy]-phenyl}-6-(4-methoxyphenyl)-1,3,5-triazin, welches durch folgende Struktur gekennzeichnet ist:

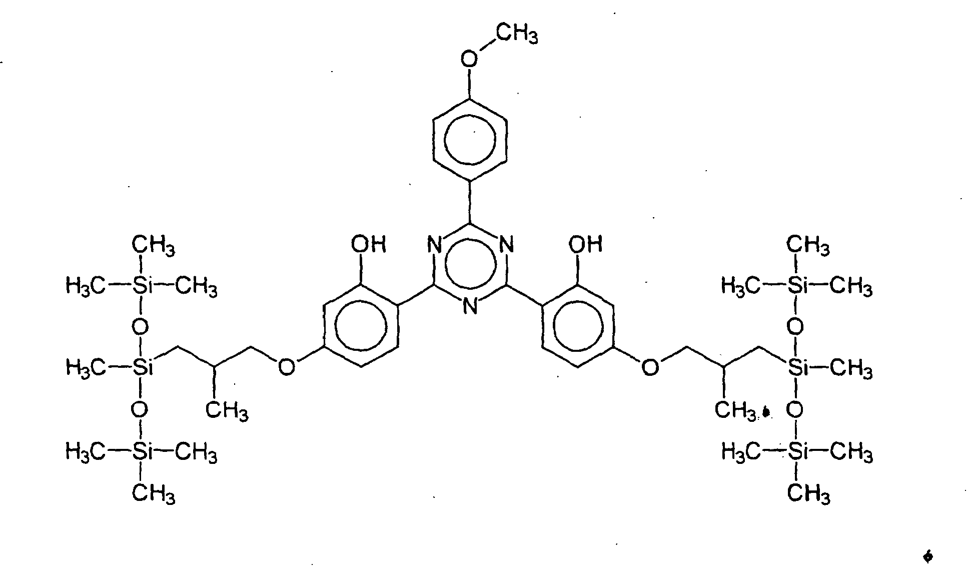

Das oder die erfindungsgemäßen unsymmetrisch substituierten s-Triazinderivate werden vorteilhaft in die Ölphase der kosmetischen oder dermatologischen Formulierungen eingearbeitet.

Ganz besonders vorteilhaft sind lichtschutzwirksame Wirkstoffkombinationen aus Polydimethyl/methyl-(3-(4,2,2-bis-ethoxycarbonylvinyl)-phenoxy-propenyl)siloxan und 2,4-Bis-{[4-(2-ethyl-hexyloxy)-2-hydroxy]-phenyl}-6-(4-methoxyphenyl)-1,3,5-triazin sowie die Verwendung von Polydimethyl/methyl-(3-(4,2,2-bis-ethoxycarbonylvinyl)-phenoxy-propenyl)siloxan zur Erzielung oder Erhöhung der Löslichkeit von 2,4-Bis-{[4-(2-ethyl-hexyloxy)-2-hydroxy]-phenyl}-6-(4-methoxyphenyl)-1,3,5-triazin.

Die Gesamtmenge an einem oder mehreren unsymmetrisch substituierten s-Triazinderivaten, insbesondere von Aniso Triazin, in den fertigen kosmetischen oder dermatologischen Zubereitungen wird vorteilhaft aus dem Bereich von 0,1 bis 15,0 Gew.-%, bevorzugt 0,5 bis 10,0 Gew.-% gewählt, bezogen auf das Gesamtgewicht der Zubereitungen.

Die erfindungsgemäßen kosmetischen oder dermatologischen Lichtschutzformulierungen können wie üblich zusammengesetzt sein und dem kosmetischen oder dermatologischen Lichtschutz, ferner zur Behandlung, Pflege und Reinigung der Haut und/oder der Haare und als Schminkprodukt in der dekorativen Kosmetik dienen.

Zur Anwendung werden die kosmetischen und dermatologischen Zubereitungen in der für Kosmetika üblichen Weise auf die Haut und/oder die Haare in ausreichender Menge aufgebracht.

Die kosmetischen und dermatologischen Zubereitungen gemäß der Erfindung können kosmetische Hilfsstoffe enthalten, wie sie üblicherweise in solchen Zubereitungen verwendet werden, z. B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als'günstige Antioxidantien alle für kosmetische und/oder dermatologische Anwendungen geeigneten oder gebräuchlichen Antioxidantien verwendet werden.

Besonders vorteilhaft werden die Antioxidantien gewählt aus der Gruppe bestehend aus Aminosäuren (z. B. Glycin, Histidin, Tyrosin, Tryptophan) und deren Derivate, Imidazole (z. B. Urocaninsäure) und deren Derivate, Peptide wie D,L-Carnosin, D-Camosin, L-Carnosin und deren Derivate (z. B. Anserin), Carotinoide, Carotine (z. B. α-Carotin, β-Carotin, Lycopin) und deren Derivate, Chlorogensäure und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol bis µmol/kg), ferner (Metall)-Chelatoren (z. B. α-Hydroxyfettsäuren, Palmitinsäure, Phytinsäure, Lactoferrin), α-Hydroxysäuren (z. B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z. B. γ-Linolensäure, Linolsäure, Ölsäure), Folsäure und deren Derivate, Ubichinon und Ubichinol und deren Derivate, Vitamin C und Derivate (z. B. Ascorbylpalmitat, Mg-Ascorbylphosphat, Ascorbylacetat), Tocopherole und Derivate (z. B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z. B. ZnO, ZnSO₄) Selen und dessen Derivate (z. B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Ölphase der Formulierungen, welche die erfindungsgemäße Stoffkombination enthalten, wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether, vorteilhaft ist z. B. Dicaprylylether.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Caprylat/Caprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Erfindungsgemäß vorteilhaft sind ferner z. B. natürliche Wachse tierischen und pflanzlichen Ursprungs, wie beispielsweise Bienenwachs, Chinawachs, Hummelwachs und andere Insektenwachse sowie Sheabutter.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Auch Moisturizer können bevorzugt verwendet werden.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Homschicht (auch transepidermal water loss (TEWL) genannt) zu reduzieren und/oder die Hydratation der Homschicht positiv zu beeinflussen.

Vorteilhafte Moisturizer im Sinne der vorliegenden Erfindung sind beispielsweise Glycerin, Milchsäure, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registratumummer 178463-23-5 abgelegt und z. B. unter der Bezeichnung Fucogel®1000 von der Gesellschaft SOLABIA S.A. erhältlich ist.

Entsprechend ihrem Aufbau können kosmetische oder topische dermatologische Zusammensetzungen im Sinne der vorliegenden Erfindung, beispielsweise verwendet werden als Hautschutzcrème, Reinigungsmilch, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Günstig sind auch solche kosmetischen und dermatologischen Zubereitungen, die in der Form eines Sonnenschutzmittels vorliegen. Vorzugsweise enthalten diese neben den erfindungsgemäßen Wirkstoffkombinationen zusätzlich mindestens eine UV-A-Filtersubstanz und/oder mindestens eine UV-B-Filtersubstanz. Solche Formulierungen können, obgleich nicht notwendig, gegebenenfalls auch ein oder mehrere anorganische Pigmente als UV-Filtersubstanzen enthalten.

Bevorzugt sind anorganische Pigmente auf Basis von Metalloxiden und/oder anderen in Wasser schwerlöslichen oder unlöslichen Metallverbindungen, insbesondere der Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxiden der entsprechenden Metalle sowie Abmischungen aus solchen Oxiden.

Auch ein zusätzlicher Gehalt an stabilisierend wirkenden Titandioxid- und/oder Zinkoxidpartikeln kann selbstverständlich vorteilhaft sein, ist aber im Sinne der vorliegenden Erfindung nicht notwendig.

Es ist auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescrèmes gewöhnlich UV-A- bzw. UV-B-Filtersubstanzen eingearbeitet.

Auch stellen UV-Schutzsubstanzen, ebenso wie Antioxidantien und, gewünschtenfalls, Konservierungsstoffe, einen wirksamen Schutz der Zubereitungen selbst gegen Verderb dar.

Vorteilhaft enthalten erfindungsgemäße Zubereitungen Substanzen, die UV-Strahlung im UV-A- und/oder UV-B-Bereich absorbieren, wobei die Gesamtmenge der Filtersubstanzen z. B. 0,1 Gew.-% bis 30 Gew.-%, vorzugsweise 0,5 bis 20 Gew.-%, insbesondere 1,0 bis 15,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Enthalten die erfindungsgemäßen Emulsionen UV-B-Filtersubstanzen, können diese öllöslich oder wasserlöslich sein. Erfindungsgemäß vorteilhafte öllösliche UV-B-Filter sind z. B.:
- 3-Benzylidencampher-Derivate, vorzugsweise 3-(4-Methylbenzyliden)campher, 3-Benzylidencampher;
- 4-Aminobenzoësäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester,
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester,
- hinsichtlich der C₃-Achse des Triazingrundkörpers symmetrisch Triazinderivate, vorzugsweise 4,4',4"-(1,3,5-Triazin-2,4,6-triyltriimino)-tris-benzoesäure-tris(2-ethylhexylester) [INCl: Octyl Triazone], welches von der BASF Aktiengesellschaft unter der Warenbezeichnung UVINUL® T 150 vertrieben wird,
- Benzotriazolderivate, vorzugsweise 2,2'-Methylen-bis-(6-(2H-benzotriazol-2-yl)-4-(1,1,3,3-tetramethylbutyl)-phenol)
- sowie an Polymere gebundene UV-Filter.

Vorteilhafte wasserlösliche UV-B-Filter sind z. B.:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxy-benzophenon-5-sulfonsäure und ihre Salze;
- Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und ihre Salze.

Eine weitere vorteilhafte UV-Filtersubstanz ist Dioctylbutylamidotriazon (INCl: Dioctylbutamidotriazone), welches unter der Handelsbezeichnung UVASORB HEB bei Sigma 3V erhältlich ist.

Die Liste der genannten UV-B-Filter, die in den erfindungsgemäßen Zubereitungen verwendet werden können, soll selbstverständlich nicht limitierend sein.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Ein vorteilhafter Breitbandfilter im Sinne der vorliegenden Erfindung ist das 2,2'-Methylen-bis-(6-(2H-benzotriazol-2-yl)-4-(1,1,3,3-tetramethylbutyl)-phenol), welches durch die chemische Strukturformel 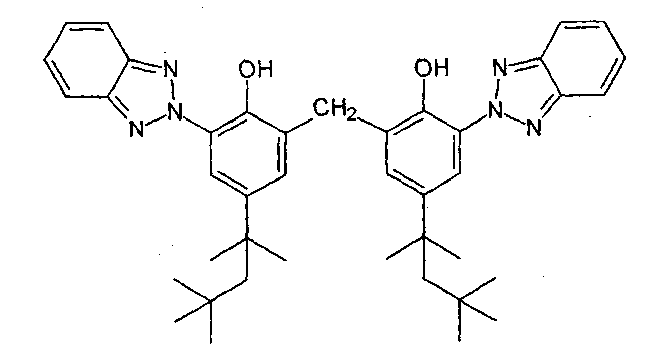 gekennzeichnet ist und unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trymethylsilyl)oxy]disiloxanyl]propyl]-phenol (CAS-Nr.: 155633-54-8) mit der INCl-Bezeichnung Drometrizole Tri-siloxane, welches durch die chemische Strukturformel 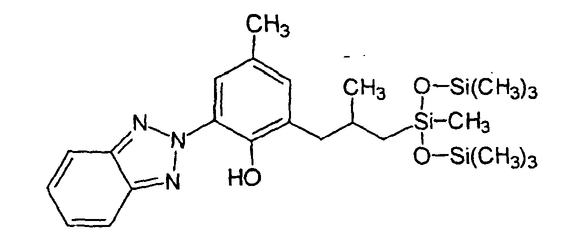 gekennzeichnet ist.

Es kann auch von Vorteil sein, in erfindungsgemäßen Zubereitungen UV-A-Filter zu verwenden, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion.

Weitere vorteilhafte UV-A-Filtersubstanzen sind die Phenylen-1,4-bis-(2-benzimidazyl)-3,3'-5,5'-tetrasulfonsäure: 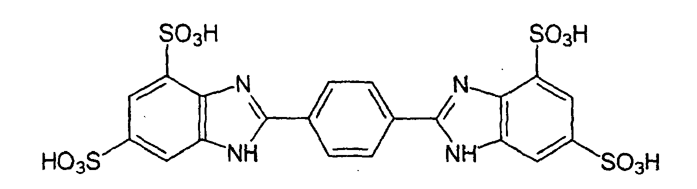 und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz: 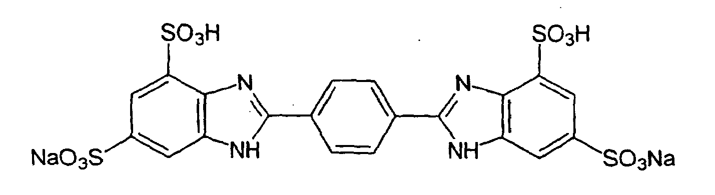 sowie das 1,4-Di(2-oxo-10-sulfo-3-bornylidenmethyl)-Benzol und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird und sich durch die folgende Struktur auszeichnet:

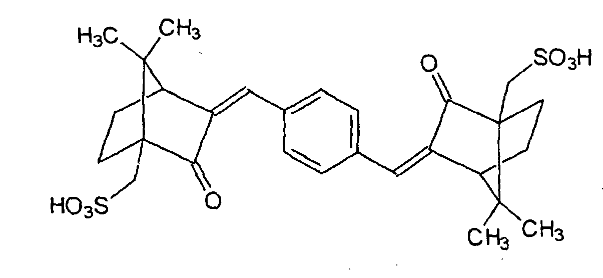

Auch Zubereitungen, die UV-A-Filter enthalten, sind Gegenstand der Erfindung. Es können die für die UV-B-Kombination verwendeten Mengen eingesetzt werden.

Ferner kann es gegebenenfalls von Vorteil sein, erfindungsgemäß weitere UV-A-und/oder UV-B-Filter in kosmetische oder dermatologische Zubereitungen einzuarbeiten, beispielsweise bestimmte Salicylsäurederivate wie 4-Isopropylbenzylsalicylat, 2-Ethylhexylsalicylat (= Octylsalicylat), Homomenthylsalicylat.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist und sich durch folgende Struktur auszeichnet:

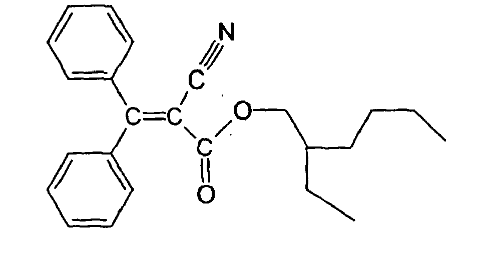

Die Liste der genannten UV-Filter, die im Sinne der vorliegenden Erfindung eingesetzt werden können, soll selbstverständlich nicht limitierend sein.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele:

| | **1** | **2** | **3** | **4** | **5** | **6** |
|---|---|---|---|---|---|---|
| | **O/W 1** | **O/W 2** | **O/W 3** | **W/O1** | **W/O 2** | **W/O 3** |
| Stearinsäure | 1,50 | 1,50 | - | - | - | - |
| Glycerinmonostearat | 3,00 | 3,00 | - | - | - | - |
| Sorbitanstearat | - | - | 3,00 | - | - | - |
| Polyglyceryl-3-methylglucose Distearat | - | - | 1,50 | - | - | - |
| Polyglyceryl-2 Dipolyhydroxystearat | - | - | - | 5,00 | - | - |
| Cetyl Dimethicon Copolyol | - | - | - | - | - | 5,00 |
| PEG-30 Dipolyhydroxystearat | - | - | - | - | 4,00 | - |
| Dimethicon | | | - | 2,00 | - | 5,00 |
| Phenyltrimethicon | 2,00 | 2,00 | - | - | 5,00 | 3,00 |
| Vitamin E-Acetat | 0,50 | 0,50 | 0,50 | - | 0,50 | - |
| Caprylic Capric Triglycerid - | 5,00 | 5,00 | | 5,00 | - | - |
| Mineralöl | - | - | - | 5,00 | 5,00 | - |
| C₁₂₋₁₅ Alkylbenzoat | - | 3,00 | 2,00 | - | - | - |
| Butylenglycol Dicaprylat/Dicaprat | - | - | 2,00 | - | - | - |
| Dicaprylyether | 3.00 | 3,00 | 5,00 | - | - | - |
| Dioctylbutamidotriazon | | - 1,00 | - | 3,00 | - | - |
| Aniso Triazin | 3,00 | 4,00 | 3,00 | 5,00 | 5,00 | 2,00 |
| Parsol SLX ® | 5,00 | 6,00 | 3,00 | 7,00 | 10,00 | 5,00 |
| Octyltriazon | - | 1,00 | - | 2,00 | - | 1,00 |
| Methylbenzyliden Campher | - | 2,00 | 4,00 | - | - | 4,00 |
| Octocrylen | - | - | 4,00 | 2,00 | - | 5,00 |
| Octylsalicylat | - | - | - | 2,00 | - | - |
| Ethylhexylmethoxycinnamat | - | | 2 | 2 | | 5 |
| TiO₂ | | 2 | | | 2 | |
| Aerosil R972 ®¹ | - | - | - | - | 0,50 | - |
| Konservierung | 0,50 | 0,50 | 0,50 | 0,50 | 0,50 | 0,50 |
| Glycerin | 3,00 | 10,00 | 10,00 | 5,00 | 10,00 | 5,00 |
| Phenylbenzimidazol Sulfonsäure | | | 3 | | | 3 |
| MgSO₄ | - | - | - | 1,00 | 1,00 | - |
| NaCl | - | - | - | - | - | 1,00 |
| Xanthan Gummi | 0,30 | 0,30 | - | - | - | - |
| Pemulen TR1 ®² | - | - | 0,10 | - | - | - |
| Natronlauge 45% | 0,50 | 0,50 | 1,20 | - | - | 1,30 |
| Wasser | ad 100,00 | ad 100,00 | ad 100,00 | ad 100,00 | ad 100,00 | ad 100,00 |

| | **7** | **8** | **9** | **10** |
|---|---|---|---|---|
| | **Hydro-dispersion** | **W/O-Pickering-emulsion** | **Spray** | **Spray** |
| Glycerinmonostearat | - | - | 4,00 | - |
| Glycerinmonostearat SE | - | - | - | 4,50 |
| Ceteareth-20 | - | - | - | 1,00 |
| Ceteareth-12 | - | - | 1,50 | - |
| Dimethicon | - | - | - | 2,00 |
| Phenyltrimethicon | 1 | 5,00 | - | - |
| Vitamin E-Acetat | - | 0,50 | - | - |
| Caprylic Capric Triglycerid | 3 | | | |
| Mineralöl | | | | 2 |
| C₁₂₋₁₅ Alkylbenzoat | 2 | | | 2 |
| Butylenglycol Dicaprylat/Dicaprat | | 5 | | |
| Octyldodecanol | | | | |
| Dicaprylyether | 2 | | | |
| Dioctylbutamidotriazon | | 2 | - | 2,00 |
| Aniso Triazin | 2,00 | 5,00 | 2,00 | 3,00 |
| Parsol SLX® | 5 | 7 | 5 | 5 |
| Octyltriazon | - | 1 | 1 | 1 |
| Methylbenzyliden Campher | - | - | - | 1 |
| Butyl Methoxydibenzoylmethan | | | | 2 |
| Octocrylen | - | 5 | - | |
| Ethylhexylmethoxycinnamat | | 5 | 5 | - |
| Eusolex T2000 ®³ | | 4,00 | - | - |
| Aerosil R972 ®¹ | - | 1,00 | - | - |
| Konservierung | 0,50 | 0,50 | 0,50 | 0,50 |
| Glycerin | 3 | 5 | 10,00 | 5,00 |
| Xanthan Gummi | 0,50 | - | - | - |
| Pemulen TR1 ®² | 0,30 | - | - | - |
| Natronlauge 45% | 0,30 | - | 0,40 | - |
| Wasser | ad 100,00 | ad 100,00 | ad 100,00 | ad 100,00 |

| | | | | |
|---|---|---|---|---|
| ¹ hydrophobierte Siliciumdioxdpigmente ² Copolymer aus C₁₀₋₃₀ Alkylacrylaten und einem oder mehreren Monomeren der Acrylsäure, der Methacrylsäure oder deren Ester (CTFA-Bez.: Acrylates/C 10-30 Alkyl Acrylate Crosspolymer) ³ hydrophobierte Titandioxdpigmente | | | | |

## Patentansprüche

1. Lichtschutzwirksame Wirkstoffkombinationen aus
(a) einer oder mehreren UV-Filtersubstanzen, gewählt aus der Gruppe
■ 2,4-Bis-{[4-(2-ethyl-hexyloxy)-2-hydroxy]-phenyl}-6-(4-methoxyphenyl)-1,3,5-triazin,
■ 2,4-Bis-{[4-(3-sulfonato)-2-hydroxy-propyloxy)-2-hydroxy]-phenyl}-6-(4-methoxyphenyl)-1,3,5-triazin Natriumsalz,
■ 2,4-Bis-{[4-(3-(2-propyloxy)-2-hydroxy-propyloxy)-2-hydroxy]-phenyl}-6-(4-methoxyphenyl)-1,3,5-triazin,
■ 2,4-Bis-{[4-(2-ethyl-hexyloxy)-2-hydroxy]-phenyl}-6-[4-(2-methoxyethyl-carboxyl)-phenylamino]-1,3,5-triazin,
■ 2,4-Bis-{[4-(3-(2-propyloxy)-2-hydroxy-propyloxy)-2-hydroxy]-phenyl}-6-[4-(ethylcarboxyl)-phenylamino]-1,3,5-triazin,
■ 2,4-Bis-{[4-(2-ethyl-hexyloxy)-2-hydroxy]-phenyl}-6-(1-methyl-pyrrol-2-yl)-1,3,5-tri-. azin,
■ 2,4-Bis-{(4-tris{trimethylsiloxy-silylpropyloxy)-2-hydroxy]-phenyl}-6-(4-methoxyphenyl)-1,3,5-triazin,
■ 2,4-Bis-{[4-(2-methylpropenyloxy)-2-hydroxy]-phenyl}-6-(4-methoxyphenyl)-1,3,5-triazin und
■ 2,4-Bis-{[4-(1',1',1',3',5',5',5'-Heptamethylsiloxy-2-methyl-propyloxy)-2-hydroxy]-phenyl}-6-(4-methoxyphenyl)-1,3,5-triazin,
und
(b) einem oder mehreren Organosiloxanen, welche sich durch die folgende Strukturformel auszeichnen: 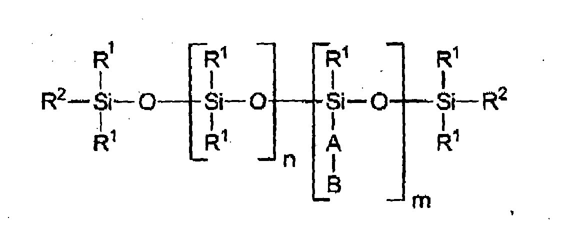
worin
◆ R¹ eine Methylgruppe und
◆ R² eine Methylgruppe oder die Gruppe A-B darstellen,
◆ n eine ganze Zahl zwischen 0 und 200 und m eine ganze Zahl zwischen 0 und 50 darstellt und
◆ die Gruppe A-B eine der beiden folgenden Strukturen hat: 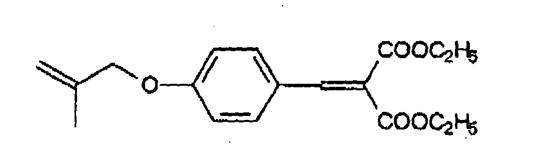 oder 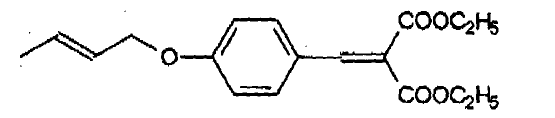 mit den Maßgaben, daß R² der Gruppe A-B entspricht, wenn m = 0 ist, und daß R² eine Methylgruppe ist, wenn m zwischen 1 und 50 liegt.

2. Wirkstoffkombinationen nach Anspruch 1, **dadurch gekennzeichnet, daß** als Organosiloxan Dimethicone-diethylbenzalmalonate gewählt wird.

3. Wirkstoffkombinationen nach einem der Ansprüche 1 oder 2, **dadurch gekennzeichnet, daß** als unsymmetrisch substituiertes Triazinderivate das Aniso Triazin gewählt wird.

4. Zubereitung, enthaltend Wirkstoffkombinationen nach einem der Ansprüche 1 bis 3, **dadurch gekennzeichnet, daß** die Gesamtmenge an einem oder mehreren unsymmetrisch substituierten s-Triazinderivaten, insbesondere von Aniso Triazin, in den fertigen kosmetischen oder dermatologischen Zubereitungen 0,1 bis 15,0 Gew.-%, bevorzugt 0,5 bis 10,0 Gew.-% beträgt, bezogen auf das Gesamtgewicht der Zubereitungen.

5. Verwendung von Wirkstoffkombinationen nach einem der Ansprüche 1 bis 3 zur Herstellung sandabweisender kosmetischer Zubereitungen.

## Claims

1. Photoprotective active ingredient combinations of
(a) one or more UV filter substances selected from the group
• 2,4-bis{[4-(2-ethylhexyloxy)-2-hydroxy]-phenyl}-6-(4-methoxyphenyl)-1,3,5-triazine,
• 2,4-bis{[4-(3-sulphonato)-2-hydroxypropyloxy)-2-hydroxy]phenyl}-6-(4-methoxyphenyl)-1,3,5-triazine sodium salt,
• 2,4-bis{[4-(3-(2-propyloxy)-2-hydroxypropyloxy)-2-hydroxy]phenyl}-6-(4-methoxyphenyl)-1,3,5-triazine,
• 2,4-bis{[4-(2-ethylhexyloxy)-2-hydroxy]-phenyl}-6-[4-(2-methoxylethylcarboxyl)phenylamino]-1,3,5-triazine,
• 2,4-bis{[4-(3-(2-propyloxy)-2-hydroxypropyloxy)-2-hydroxy]phenyl}-6-[4-(ethylcarboxyl)phenylamino]-1,3,5-triazine,
• 2,4-bis-{[4-(2-ethylhexyloxy)-2-hydroxyl]-phenyl}-6-(1-methylpyrrol-2-yl)-1,3,5-triazine,
• 2,4-bis{[4-tris(trimethylsiloxysilylpropyloxy)-2-hydroxyl]phenyl}-6-(4-methoxyphenyl)-1,3,5-triazine,
• 2,4-bis{[4-(2-methylpropenyloxy)-2-hydroxy]-phenyl}-6-(4-methoxyphenyl)-1,3, 5-triazine and
• 2,4-bis{[4-(1',1',1',3',5',5',5'-heptamethylsiloxy-2-methylpropyloxy)-2-hydroxy]phenyl}-6-(4-methoxyphenyl)-1,3,5-triazine
and
(b) one or more organosiloxanes which are **characterized by** the following structural formula: 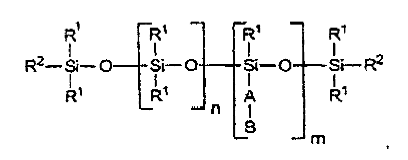
in which
• R¹ is a methyl group and
• R² is a methyl group or the group A-B,
• n is an integer between 0 and 200 and m is an integer between 0 and 50 and
• the group A-B has one of the two following structures: 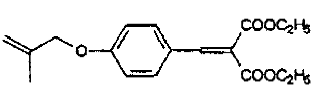 or 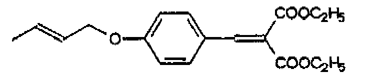 with the provisos that R² corresponds to the group A-B when m = 0, and that R² is a methyl group when m is between 1 and 50.

2. Active ingredient combinations according to Claim 1, **characterized in that** dimethicone diethylbenzalmalonate is selected as organosiloxane.

3. Active ingredient combinations according to one of Claims 1 or 2, **characterized in that** aniso triazine is selected as asymmetrically substituted triazine derivative.

4. Preparation comprising active ingredient combinations according to one of Claims to 3, **characterized in that** the total amount of one or more asymmetrically substituted s-triazine derivatives, in particular of aniso triazine, in the finished cosmetic or dermatologic preparations is 0.1 to 15.0% by weight, preferably 0.5 to 10.0% by weight, based on the total weight of the preparations.

5. Use of active ingredient combinations according to one of Claims 1 to 3 for producing sand-repellent cosmetic preparations.

## Revendications

1. Combinaisons de substances actives en protection contre la lumière, constituées par
(a) une ou plusieurs substances filtres des UV, choisies dans le groupe formé par
- 2,4-bis-{[4-(2-éthylhexyloxy)-2-hydroxy)-phényl}-6-(4-méthoxyphényl)-1,3,5-triazine
- sel sodique de la 2,4-bis-{[4-(3-sulfonato)-2-hydroxypropyloxy)-2-hydroxy]-phényl}-6-(4-méthoxyphényl)-1,3,5-triazine
- 2,4-bis-{[4-(3-(2-propyloxy)-2-hydroxypropyloxy)-2-hydroxy]-phényl}-6-(4-méthoxyphényl)-1,3,5-triazine
- 2,4-bis-{[4-(2-éthylhexyloxy)-2-hydroxy]-phényl}-6-[4-(2-méthoxyéthylcarboxyl)-phénylamino]-1,3,5-triazine
- 2,4-bis-{[4-(3-(2-propyloxy)-2-hydroxypropyloxy)-2-hydroxy]-phényl}-6-[4-(éthylcarboxyl)-phénylamino]-1,3,5-triazine
- 2,4-bis-{[4-(2-éthylhexyloxy)-2-hydroxy]-phényl}-6-(1-méthylpyrrol-2-yl)-1,3,5-triazine
- 2,4-bis{[4-tris(triméthylsiloxysilylpropyloxy)-2-hydroxy]phényl}-6-(4-méthoxyphényl)-1,3,5-triazine
- 2,4-bis-([4-(2-méthylpropényloxy)-2-hydroxy]phényl}-6-(4-méthoxyphényl)-1,3,5-triazine et
- 2,4-bis-{[4-(1',1',1',3',5',5',5'-heptaméthylsiloxy-2-méthylpropyloxy)-2-hydroxy]-phényl}-6-(4-méthoxyphényl)-1,3,5-triazine et
(b) un ou plusieurs organosiloxanes, qui sont **caractérisés par** la formule de structure suivante : 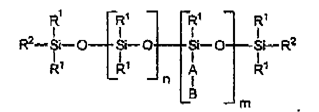
où
R¹ représente un groupe méthyle et
R² représente un groupe méthyle ou le groupe A-B
n vaut un nombre entier entre 0 et 200 et m vaut un nombre entier entre 0 et 50 et
le groupe A-B présente une des deux structures suivantes : 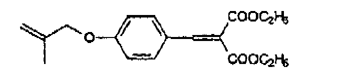 ou 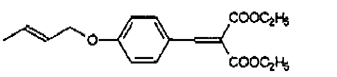 à condition que R² corresponde au groupe A-B, lorsque m = 0, et que R² représente un groupe méthyle lorsque m est situé entre 1 et 50.

2. Combinaisons de substances actives selon la revendication 1, **caractérisées en ce qu'**on choisit le diméthicone-benzalmalonate de diéthyle comme organosiloxane.

3. Combinaisons de substances actives selon l'une quelconque des revendications 1 ou 2, **caractérisées en ce qu'**on choisit l'anisotriazine comme dérivé de triazine substituée de manière non symétrique.

4. Préparation contenant des combinaisons de substances actives selon l'une quelconque des revendications 1 à 3, **caractérisée en ce que** la quantité totale d'un ou de plusieurs dérivés de s-triazine, substitués de manière non symétrique, en particulier d'anisotriazine, dans les préparations cosmétiques ou dermatologiques finies est de 0,1 à 15,0% en poids, de préférence de 0,5 à 10,0% en poids par rapport au poids total des préparations.

5. Utilisation de combinaisons de substances actives selon l'une quelconque des revendications 1 à 3 pour la préparation de préparations cosmétiques repoussant le sable.
